# EUROPEAN PATENT SPECIFICATION

(11) **EP 0 772 446 B1**
(45) Date of publication and mention of the grant of the patent: **23.10.2002**
(21) Application number: 95926564.6
(22) Date of filing: 13.07.1995
(51) Int. Cl.: A61K 31/727, A61P 41/00

(54) **ANTI-ADHESION AGENT**
ANTI-ADHÄSIONSMITTEL
AGENT ANTI-ADHERENCE

(30) Priority: 19.07.1994 SE 9402529
(43) Date of publication of application: 14.05.1997
(73) Proprietor: Medicarb AB, 168 65 Bromma (SE)
(72) Inventor: HANSSON, Hans-Arne, 436 58 Hovas (SE); JOHANSSON-RUDEN, Gunilla, 436 42 Askim (SE); LARM, Olle, 169 39 Bromma (SE)
(74) Representative: Giver, Sören Bo
(86) International application number: SE9500856
(87) International publication number: WO96002258

(56) References cited:
- US-A- 4 326 532
- US-A- 5 116 824
- JOURNAL OF PERIODONTOLOGY, Volume 62, No. 10, 1991, S. PITARU et al., "Heparan Sulfate and Fibronectin Improve the Capacity of Collagen Barriers to Prevent Apical Migration of the Junctional Epithelium", page 598.

## Description

### Technical area

The present invention relates to new anti-adhesion agents, i.e. products having the ability to prevent non-desired adhesion of tissues in connection with wound healing. The product has also the ability to improve healing quality by stimulating regeneration of tissue.

The invention also includes a process to prevent such undesired adhesion of tissues.

### Background of the invention

Our ability to move freely as desired and according to the current need is of utmost importance for our life quality. Suitable function of the musculoskeletal system in close cooperation with mainly the skin, mucosal membranes and nervous tissue is a prerequisite for our ability to move and requires per se that different structures such as bone, muscles and tendons are freely moveable relative to each other. Such activities require sliding zones, minimal friction and maximized freedom to move. Sliding systems between for example adjacent muscles and tendons as well as between skin and adjacent tissues are thus required for optimal function. The same is true for visceral structures, such as the gastrointestinal tract, heart, lung, brain and spinal cord. The sliding systems are formed by thin sheets of loose connective tissue, which in the abdomen, thorasic cavity, pericardial space and for the brain and spinal cord are delimited by mesothelial cells. The paratenons show a similar design.

These sliding systems are very sensitive to inflammation and injury. Scar tissues are easily formed resulting in impaired function and even loss of function. Adhesions may be formed in the abdominal cavity, i.e. formation of strings, and membrane-like fusions of adjacent or surrounding structures may eventually result in ileus, a life threatening condition. Surgical procedures carried out to treat injuries, remove tumors or treat other disorders or for performing reconstructions always result in scar formation and thereby more or less comprehensive loss of "natural and original" sliding systems.

The healing of injuries to the skin and mucosal linings are complicated, on the one hand by the restricted ability of connective tissue to regenerate and on the other hand by the formation of immature granulation tissue, which latter has a limited capacity to gain a maturity similar to that of normal tissue. Thus, the dermis is neither reformed in youths nor in adults after injury with few exceptions. Small and/or superficial injuries to the dermis are healed by replacement of the lost tissue by generation of adjacent type like structures and by the generation of reactive granulation tissue. More extensive tissue losses, such as after deep burns, third degree thermal injuries, and after loss of part of the dermis inevitably heal with scar formation, variable but persistent loss of tissue and with permanent deformations. The mechanically resistant component in the scar tissue is largely constituted by the type III collagen of short fibres and inferior organisation and has therefore inferior mechanical properties as compared to collagen of the normal optimal type I. The fraction of amorphous plastic ground substance has been reduced as well as the tissue cellularity. The number of blood vessels is with time reduced in relation to that in normal tissue and the distribution and type of vessels have changed. Wide, thin-walled vessels of inferior function as compared to the corresponding normal blood vessels are frequent, as are abnormal lymph vessel systems. The sliding systems are thus eventually replaced by rigid, fibrous collagen connective tissue.

An additionally complicating, very important factor is created by the appearance of *myofibroblasts*, i.e. "common" connective tissue cells (fibroblasts) which, as well as a part of the macrophages, have an increased number of cytoplasmic bundles of muscle proteins which enable the cells to slowly and powerfully contract and maintain the contraction for a long period of time. This may result in contractures which further deform and limit the function of the affected tissue. An increased presence of myofibroblasts is seen for example around breast implants (silicon prosthesis implanted for breast enlargement or breast reconstruction; a more detailed description by C. Lossing & H-A Hansson is found in an article in Plastic Reconstr.Surgery 1993, Vol. 91, page 1277-1286) and around sutures and other implants of foreign materials. Myofibroblasts are prevailing at an increased frequency around joints in certain rheumatoid diseases and may result in deviation of fingers and sometimes also in luxations. This cell is the pathogenic factor causing the deformaties of the hand striking patients with Dupuytren's contracture. The myofibroblasts as well as common fibroblasts are attached to collagen threads by means of specific heterodimeric receptors, one unit of which always is constituted by *β1-integrin*. Blocking of integrins results in elimination of contractures. Inflammation reducing drugs may influence the expression of integrins.

Sliding systems in loose connective tissue with or without a well defined sliding surface thus only restores in cases with minimal inflammation. The formation of granulation tissue occur, however, only in conjunction with an inflammatory process which per se does result in formation of immature cells and tissue components. This inability of the new tissue formed during the repair process to approach the normal differentiation levels is the reason for that scar tissue is of inferior quality quantitatively and qualitatively as compared to the original, mature but lost tissue. Maturation of regenerated tissue requires access to growth factors which control and promote differentiation of cells, fibres and ground substance.

### Background art

Extensive research has been directed to solve the problem of avoiding undesired adhesion of tissues in connection with the healing of wounds, for example wounds caused by surgical incisions, by accident, inflammations and tumors. PCT application No. US90/02406 describes technology associated with this specific problem and also includes a relatively extensive elucidation of the background art. The techniques described in said patent application is based on the use of sandwich constructions comprising a biodegradable bioactive membrane, the opposed surfaces of which have different composition and thereby different biological functions. However, the corresponding products do not seem to be available on the market.

### Summary of the invention

Accordingly, the present invention has for an object to provide an anti-adherence agent with the use of which there is induced only a minimal inflammation of short duration, said agent being, moreover, bioacceptable and biodegradable without resulting in interfering degradation products.

Another object of the invention is to provide an anti-adherence agent having the ability to induce interface surfaces and resulting in simplified mechanical and technical handling in connection with for example surgical incisions.

Yet another object of the invention is the use of chitosan and a polysaccharide immobilized thereto selected from heparin, heparan sulphate and dextran sulphate for the manufacture of a medicament for prevention or substantial reduction of undesirable adhesion of damaged tissue with adjacent or surrounding tissues in connection with wound healing.

A further object of the invention is to stimulate regeneration of tissue in connection with wound healing.

For these and other objects which will be elucidated by the following disclosure there is provided through the present invention a new use of chitosan and a polysaccharide immobilized thereto, said polysaccharide being selected from heparin, heparan sulphate and dextran sulphate. While using this composition of matter there can be produced an agent having the ability to eliminate or substantially reduce undesirable nt having the ability to eliminate or substantially reduce undesirable adhesion of damaged tissue with adjacent or surrounding tissues in connection with wound healing.

The polysaccharide used can be immobilized to the chitosan in mainly three different ways. Thus, immobilization can take place by ionic binding, by covalent binding or by mechanical inclusion in the chitosan in connection with precipitation from solution. A process for covalent binding of the relevant polysaccharide to a substrate carrying amino groups is described in US patent No. 4,613,665.

As a polysaccharide it is particularly preferred to use heparin or heparan sulphate said substances being commercially available on the market from several manufacturers. Also partly hydrolyzed forms of the polysaccharide can, of course, be used provided that the biological activity is maintained.

The anti-adherence agent used in accordance with the invention can be present in different physical forms, for example as films or membranes, gels, tubes or hoses, powders, aerosols or solutions. The relevant form is, of course, adapted to the damage involved. In most cases films are useful, whereas tubes or gels can be used in special cases, for example in connection with elongate confined tissues, such as muscles and tendons.

Chitosan is a linear 1,4-bound polysaccharide built up from β-D-glucose amine units. The chitosan is manufactured by N-deacetylation of chitin, a polymer forming the shell of inter alia insects and shellfish. Commercially, chitin is recovered from crab and shrimp shells which constitute waste products from the fishing industry. By controlling the alkaline treatment of chitins chitosans of varying degree of N-acetylation can be made. When treating chitin with concentrated alkali, usually sodium hydroxide, N-deacetylation thus takes place, i.e. acetamido groups are converted into amino groups to form chitosan.

The physical properties of chitosan affecting its usefulness depend on the degree of N-acetylation, the molecular weight and the homogeneity. Chitosan is biodegradable, both by chitinase in the digestive system and by lysozyme and other enzymes in the body liquids.

It is preferred in connection with the use of the present invention that the chitosan has a degree of N-acetylation of at most about 90% and preferably at most about 50%. It is particularly preferred that the degree of N-acetylation is less than about 25%.

The present invention also provides for a process to prevent or substantially reduce undesireable adhesion of tissues in connection with wound healing. This process involves applying at the site of the wound healing an agent comprising chitosan and a polysaccharide immobilized thereto selected from heparin, heparan sulphate and dextran sulphate.

Depending on the character of the wound involved the agent can be applied in the form of a film, in the form of a gel or in the form of a tube or a hose. The product to be selected for the application can easily be decided in connection to for example the relevant surgical procedure.

### Examples of preferred embodiments

The present invention will in the following be illustrated in connection with nonlimiting examples. In the examples all films have been prepared in Petri dishes having a surface area of 54 cm².

### EXAMPLE 1

### Preparation of chitosan film

5 g hydrochloride salt of chitosan (50% degree of acetylation, Pronova) are dissolved in distilled water (0.5 L, 1% v/w). 10 mL of the solution obtained are transferred to a Petri dish, and a film of chitosan is formed by evaporation and drying in a heating cabinet at 70°C for 24 h. The film obtained is then neutralized by the addition of a sodium phosphate buffer, 0.2 M, pH 9.0. The film is allowed to remain in the Petri dish in said buffer at room temperature for 2-4 h, is then washed 3-4 times with water and allowed to dry.

### EXAMPLE 2

### Preparation of chitosan film

5 g hydrochloride salt of chitosan (20% degree of acetylation, Pronova) are dissolved in a 2% acetic acid solution (0.5 L, 1% v/w). The solution is autoclaved for 1 h at 125°C for sterilization purposes. After cooling a film is made in a Petri dish, in this case with the use of 20 mL of the solution. The film is then allowed to dry at room temperature and neutralized by the addition of a sodium phosphate buffer, 0.2 M, pH 9.0, added to the dish. The film is allowed to stay in this buffer for 2-4 h at room temperature, is then washed with distilled water 3-4 times and again allowed to dry.

### EXAMPLE 3

### Nitrous acid degradation of heparin

One gram of heparin is dissolved in 300 mL of water . The solution is cooled to 0°C in ice water and maintained cold. First 10 mg of sodium nitrite (NaNO₂) is added. Then 2 mL of acetic acid is added to the solution while stirring. The reaction mixture is maintained at 0°C for two hours, dialyzed, and feeze dried. The yield is 0,7 g degraded heparin.

### EXAMPLE 4

### Periodate oxidation of heparin

A solution of sodiumperiodate-oxidized sodiumheparin is prepared in the following manner. One gram of sodiumperiodate, NaIO₄, is dissolved in 200 mL of distilled water. Ten grams of sodiumheparin is added to the solution of sodiumperiodate and is stirred over night in the dark. The resulting solution, after adding 10 mL of glycerol and stirring for two hours, is dialyzed against water. The water is exchanged every hour. This results in a solution containing periodate-oxidized heparin in a concentration of about 19 mg/mL.

### EXAMPLE 5

### Preparation of chitosan film with covalently bonded heparin (end-point attachment)

To a neutralized chitosan film prepared in accordance with Example 1 there are added 20 mL of a solution containing 125 mg nitrite degraded heparin, prepared as in Example 3, dissolved in 0.5 L water and containing 4.4 g NaCl. To the solution is added 15 mg sodium cyanoborohydride. The pH of the solution is adjusted to 3.9 using 0.5 M hydrochloric acid or another acid. The solution containing the chitosan film is allowed to stand at room temperature for 14 h, and the treated film is then washed 3-4 times with water and is allowed to dry.

### EXAMPLE 6

### Preparation of chitosan film with covalently bonded heparin (multi point attachment)

A neutralized chitosan film prepared in accordance with Example 2 is allowed to stay for 24 h in 20 mL of the following solution.
4,4 sodiumchloride and 125 mg periodate oxidized heparin, prepared as described in Example 4, are dissolved in 0.5 L of water, the pH is adjusted to 3.9 using 0.5 M hydrochloric acid. To the solution there is added 15 mg sodium cyanoborohydride, and the solution is kept for 10 hours at room temperature. The treated film is then washed with water 3-4 times and allowed to dry. With regard to details concerning this technique of covalent binding of heparin reference is made to the above-mentioned US patent No. 4,613,665.

### EXAMPLE 7

### Preparation of chitosan film with ionically bonded heparin.

A neutralized chitosan film is prepared as in Example 2. A solution of heparin (125 g in 0,5L water containing 4.4 g NaCl) is added. After 3 hours at room temperature the film is rinsed with 2x 0.5 L water and dried.

### EXAMPLE 8

### Biological test, control

The film prepared in accordance with Example 2 is used as an anti-adherence membrane in the following animal model.
The abdominal wall of a rat is opened and on each side of the sagittal line there is produced in a surgical manner a wound about 12x10 mm. One defect is covered with a film from Example 2, a piece of about 18x15 mm, whereas as the other defect is left open. The membrane is sutured using Dexon ® 7-0 in such a manner that no suture is exposed in the abdominal cavity.

The result is evaluated after 2 and 4 week. In this connection modest adherences in the abdominal cavity against the membrane are observed, whereas massive adherences could be demonstrated if the tissue defect is not covered by a film.

The abdominal defect beneath the film heals essentially with scar tissue formation, and there are signs of inflammatory reaction and capsule formation around the film.

### EXAMPLE 9

### Biological test, in accordance with the invention

The film made in accordance with Example 3 is used as an anti-adherence membrane in the following animal model.

The abdominal wall of a rat is opened and on each side of the sagittal line there is created in a surgical manner a wound of about 12x10 mm.

One defect is covered with film, about 18x15 mm, whereas the other defect is left open. The membrane is sutured in the same manner as in Example 8.

The wound area left open displayed several adherences in contrast to the wound covered by the film, which had very few if any adherences.

### EXAMPLE 10

### Preparation of chitosan film with ionically bonded heparin

5 g hydrochloride salt of chitosan (45% degree of acetylation, Pronova) are dissolved in water (0.5 L, 1% v/w). The solution is autoclaved for 1 h at 125°C for sterilization purposes. After cooling a film is made in a Petri dish, in this case with the use of 20 mL of the solution. The film is then allowed to dry at room temperature and a solution of heparin (125 g in 0,5L water) is added. After 3 hours at room temperature the film is rinsed with 2x 0.5 L water and dried.

### EXAMPLE 11

### Preparation of chitosan film

5 g hydrochloride salt of chitosan (45% degree of acetylation, Pronova) are dissolved in water (0.5 L, 1% v/w). The solution is autoclaved for 1 h at 125°C for sterilization purposes. After cooling a film is made in a Petri dish, in this case with the use of 20 mL of the solution. The film is then allowed to dry at room temperature and neutralized by the addition of a sodium phosphate buffer, 0.2 M, pH 9.0, added to the dish. The film is allowed to stay in this buffer for 2-4 h at room temperature, is then washed with distilled water 3-4 times and again allowed to dry.

### EXAMPLE 12

### Biological test, in accordance with the invention

Films prepared from chitosan-heparin as described above in Example 10 are positioned to cover wounds (10 x 12 mm, depth 1 mm) prepared on the parietal abdominal wall as described above. An identical wound is prepared on the contralateral side of the abdominal wall, and covered by a Chitosan film as described in Example 11. The occurrence of adherence formation is evaluated after 2 weeks. The wound covered by the heparin-chitosan film lacks adherences while that covered by the plain chitosan film shows a few, minor adherences. Light microscopic examination of the heparin-chitosan film reveals improved healing of the wound, including the extent of covering by mesothelial-like cells, and that there is a less extensive infiltration of inflammatory cells at the interface between the heparin-chitosan film and the wounded abdominal wall tissue than in corresponding area covered by the plain chitosan film.

### EXAMPLE 13

### Preparation of chitosan-heparin films

5 g hydrochloride salt of chitosan (16% degree of acetylation, Pronova) are dissolved in a 2% acetic acid solution (0.5 L, 1% v/w). The solution is autoclaved for 1 h at 125°C for sterilization purposes. After cooling a film is made in a Petri dish, in this case with the use of 20 mL of the solution. The film is then allowed to evaporate in an oven at 70°C for 16 h. The film is treated with a 0.1 M NaOH solution for 3 h at room temerature and then washed with distilled water 3-4 times and again allowed to dry in 70°C for 2 h. The resulting film is then transferred to a Petri dish and 30 mL of a sterile solution of native heparin(1% w/v, pig mucosa, Kabivitrum) in 0.2 M fosfate buffer (pH 6.4) is added. The film is kept at room temperature over night and then rinsed with sterile water and dried in a LAF bench. Four more films are prepared as above with the modification that they are treated with 0.5%, 0.1%, 0.01% and 0.00% solutions of heparin respectively. The heparinised films are subjected to elemental analysis which shows that the films contain 1.2%, 0.9%,1.3%, 0.23 and 0.007% sulfur, respectively. These values correspond to a heparin content of 9.2%, 7.7%, 10.8%, divided into six groups with ten biopsies 1.9% and 0% respectively.

### EXAMPLE 14

### Preparation of in vitro wounds

Sterile human skin is obtained from masectomy specimens. In each experiment only skin from a single donor is used. Under sterile conditions, circles with a diameter of 6mm are cut with a biopsy punch (Stiefel Laboratories, UK). In the centre of each piece, on the epidermal side, a partial wound is made with a 3mm biopsy punch and subsequently pieces are transferred to 12-well plates (Costar) with the epidermal side up. Each well is filled with Dulbeccos Modified Eagles Medium (DMEM) to the epidermal level keeping the wound in the gas/liquid interface. Fetal Calf serum, 2% (FCS) and antibiotics (penicillin 50 µg/mL an streptomycin 50 µg/mL) are added to all samples.

### EXAMPLE 15

### In vitro healing test

The invitro wounds, described in Example 14, are divided into five groups with ten biopsies in each group. Every wound is covered with a heparinised film as decribed in Example 13. The media are changed everv day. After seven days the pieces are fixed in 4% neutral buffered formaldehyde, dehydrated through an ethanol-xylene series and embedded in paraffin. Cross sections, 10-20 mm in thickness are stained with haematoxylin and eosin and the degree of re-epithelialisation is assed by light microscopy. Only wounds totally covered with keratinocytes are regarded as healed.

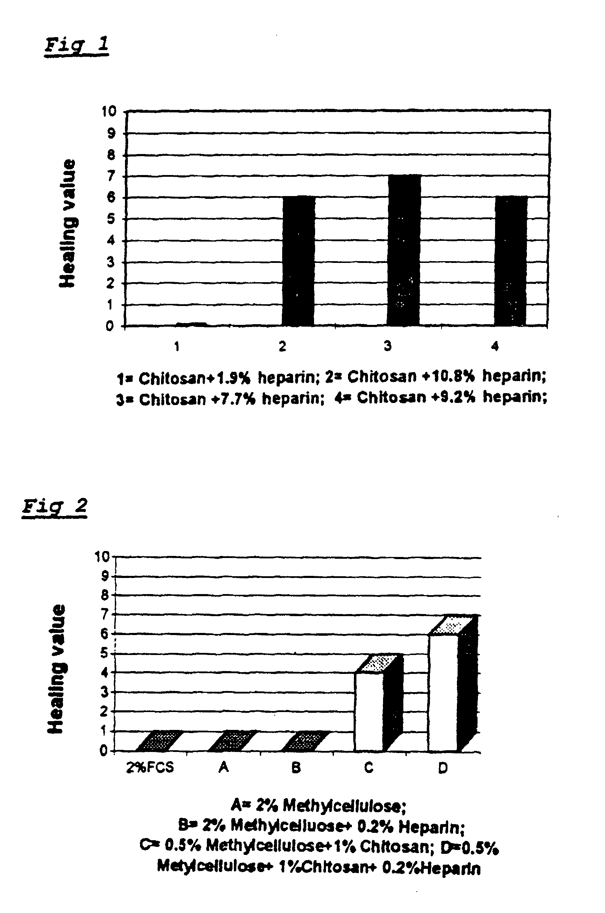

As is evident from fig 1, films with a heparin content below 2% do not stimulate cellproliferation.

### EXAMPLE 16

### Preparation of gel compositions

Water containing 0.9% NaCl is used to prepare the following four gel compositions:
A=2% Methylcellulose
B=2% Methylcellulose + 0.2% Sodiumheparin
C=0.5% Methylcellulose + 1% Chitosan (16% acetylation)
D=0.5% Methylcellulose + 1% Chitosan (16% acetylation) + 0.2 % Sodiumheparin

### EXAMPLE 17

### In vitro healing test

The invitro wounds, described in Example 14, are divided into six groups with ten biopsies in each group. Every wound in five of the groups are covered with a gel composition as decribed in Example 16. The last group is only treated with the media (2% FCS). The media are changed every day. After seven days the pieces are fixed in 4% neutral buffered formaldehyde, dehydrated through an ethanol-xylene series and embedded in paraffin. Cross sections, 10-20 mm in thickness are stained with haematoxylin and eosin and the degree of re-epithelialisation is assed by light microscopy. Only wounds totally covered with keratinocytes are regarded as healed.

As is evident from fig 2, a gel consisting of a combination of Chitosan and Heparin heals the wounds better than gels with only Chitosan or only Heparin does.

### EXAMPLE 18

### Biological test, in accordance with the invention

Example 9 is repeated using the film made in accordance with Example 7.

As is clear from the biological experiments described above the use of the techniques according to the present invention allows substantially improved healing properties in view of prevented adhesion and stimulated growth. The invention is not restricted to the examples given above and the scope of the invention is limited only by the scope of the appended claims.

With regard to the application of the invention there can be mentioned that films or membranes, gels or powder prepared as above or solutions can be used in connection with wounds and defects in or on the following organs and structures: abdominal wall; thorax wall; lung; heart-pericardium; central vessels; intestinal tract; urogenital tract; scull; cerebral meninges; spinal cord; tendons; nerves; muscles; bone; mucosa; cornea, skin etc.

Products in the form of tubes or hoses or gels can be used as guides in stimulated growth and concurrently gliding surfaces can be maintained by the fact that adhesion to the environment is avoided. Such products can be used in connection with nerves, tendons and ligaments, intestinal tract, urogenital tracts, blood vessels etc.

Even better stimulation of healing quality can probably be achieved by a combination of this invention with growth factors.

## Claims

1. The use of chitosan and a polysaccharide immobilized thereto selected from heparin, heparan sulphate and dextran sulphate for the manufacture of a medicament for prevention or substantial reduction of undesirable adhesion of damaged tissue with adjacent or surrounding tissues in connection with wound healing.

2. The use according to claim 1, wherein the polysaccharide is immobilized to the chitosan by means of ionic bonds.

3. The use according to claim 1, wherein the polysaccharide is immobilized to the chitosan by means of covalent bonds.

4. The use according to any one of the preceding claims, wherein the polysaccharide is heparin or heparan sulphate.

5. The use according to any one of the preceding claims, wherein the medicament is in the form of a film or membrane.

6. The use according to any one of the preceding claims, wherein the medicament is in the form of a tube or a hose.

7. The use according to claim 6, wherein the medicament is in the form of a gel.

8. The use according to claim 6, wherein the medicament agent is in the form of a powder, an aerosol or a solution.

9. The use according to any one of the preceding claims, wherein the chitosan has a degree of N-acetylation of at most 90% and preferably at most 50%.

## Patentansprüche

1. Verwendung von Chitosan und einem darauf immobilisierten Polysaccharid, das gewählt ist aus Heparin, Heparansulfat und Dextransulfat, für die Herstellung eines Medikaments zur Verhinderung oder erheblichen Verringerung eines unerwünschten Haftens von beschädigtem Gewebe an benachbartem oder umgebendem Gewebe in Verbindung mit einer Wundheilung.

2. Verwendung nach Anspruch 1, worin das Polysaccharid an dem Chitosan mittels ionischer Bindungen immobilisiert ist.

3. Verwendung nach Anspruch 1, worin das Polysaccharid an dem Chitosan mittels kovalenter Bindungen immobilisiert ist.

4. Verwendung nach einem der vorhergehenden Ansprüche, worin das Polysaccharid Heparin oder Heparansulfat ist.

5. Verwendung nach einem der vorhergehenden Ansprüche, worin das Medikament in Form eines Films oder einer Membran vorliegt.

6. Verwendung nach einem der vorhergehenden Ansprüche, worin das Medikament in Form eines Rohrs oder eines Schlauchs vorliegt.

7. Verwendung nach Anspruch 6, worin das Medikament in Form eines Gels vorliegt.

8. Verwendung nach Anspruch 6, worin das Medikament-Mittel in Form eines Pulvers, eines Aerosols oder einer Lösung vorliegt.

9. Verwendung nach einem der vorhergehenden Ansprüche, worin das Chitosan einen Grad der N-Acetylierung von höchstens 90 % und vorzugsweise von höchstens 50 % hat.

## Revendications

1. Utilisation de chitosane et d'un polysaccharide immobilisé sur celui-ci et choisi parmi l'héparine, l'héparane-sulfate et le dextrane-sulfate pour la fabrication d'un médicament destiné à la prévention ou la réduction substantielle de l'adhérence indésirable d'un tissu lésé avec les tissus adjacents ou environnants dans le cadre d'une cicatrisation.

2. Utilisation selon la revendication 1, dans laquelle le polysaccharide est immobilisé sur le chitosane au moyen de liaisons ioniques.

3. Utilisation selon la revendication 1, dans laquelle le polysaccharide est immobilisé sur le chitosane au moyen de liaisons covalentes.

4. Utilisation selon l'une quelconque des revendications précédentes, dans laquelle le polysaccharide est l'héparine ou l'héparane-sulfate.

5. Utilisation selon l'une quelconque des revendications précédentes, dans laquelle le médicament se présente sous la forme d'un film ou d'une membrane.

6. Utilisation selon l'une quelconque des revendications précédentes, dans laquelle le médicament se présente sous la forme d'un tube ou d'un tuyau.

7. Utilisation selon la revendication 6, dans laquelle le médicament se présente sous la forme d'un gel.

8. Utilisation selon la revendication 6, dans laquelle l'agent médicamenteux se présente sous la forme d'une poudre, d'un aérosol ou d'une solution.

9. Utilisation selon l'une quelconque des revendications précédentes, dans laquelle le chitosane possède un degré de N-acétylation au maximum de 90% et de préférence au maximum de 50%.
